# EUROPÄISCHE PATENTSCHRIFT

(11) **EP 0 880 953 B1**
(45) Veröffentlichungstag und Bekanntmachung des Hinweises auf die Patenterteilung: **01.10.2003**
(21) Anmeldenummer: 98108773.7
(22) Anmeldetag: 14.05.1998
(51) Int. Cl.: A61F 13/00, A61M 27/00, A61M 1/00, A61M 35/00

(54) **Vorrichtung zur Applikation von Wirkstoffen an einer Wundoberfläche**
Device for the application of active agents to a wound surface
Dispositif pour l'application d'agents actifs sur la surface d'une blessure

(30) Priorität: 27.05.1997 DE 19722075; 30.08.1997 DE 29715634 U
(43) Veröffentlichungstag der Anmeldung: 02.12.1998
(73) Patentinhaber: Fleischmann, Wilhelm, Dr. med., 89182 Bernstadt (DE)
(72) Erfinder: Fleischmann, Wilhelm, Dr. med., 89182 Bernstadt (DE)
(74) Vertreter: Patentanwälte Westphal, Mussgnug & Partner

(56) Entgegenhaltungen:
- WO-A-94/20041
- DE-A- 4 111 122
- GB-A- 1 549 756
- US-A- 4 382 441

## Beschreibung

Die Erfindung betrifft eine Vorrichtung zur Applikation von Wirkstoffen an einer Wundoberfläche gemäß dem Oberbegriff des Anspruchs 1.

Zur medikamentösen lokalen 3ehandlurg von Wunden werden bislang Salben, Lösungen oder feste Medikamententräger, wie resorbierbare Kollagene, antibiotikumgetränkter Knochenzement oder imprägnierte Wundauflagen, verwendet. Insbesondere bei tieferen Wunden besteht die Schwierigkeit, daß Salben nicht anwendbar sind, Lösungen von Verbandsstoffen aufgesaugt werden und häufig nicht in genügendem Maße mit der inneren Wundoberfläche in Kontakt kommen, um ihre therapeutische Wirkung zu entfalten. Bei der Implantation von körperfremden Arzneimittelträgern, wie Knochenzement oder resorbierbaren Materialien, entsteht ein Wirkungsverlust durch Verdünnung mit Wundsekret. Außerdem stehen nur wenige Arzneimittel zur Verfügung, die eine lokale Langzeit-Depotwirkung entfalten. Im Gewebe kann unter Umständen eine schädliche Fremdkörperreaktion entstehen. Abbaubare Medikamententräger können bei ihrem Zerfall zu unerwünschten Nebenwirkungen führen.

Aus der DE 40 12 232 A1 ist ein Redon- bzw. Instillationsverband für die Behandlung oberflächlicher und tiefer Problemwunden, insbesondere mit Infektionen, bekannt, bei welchem die Wunde durch eine dünne semipermeable Folie abgedeckt wird. Eine Zuleitung und eine Ableitung führen unter die Folie in den Wundbereich. Über die Zuleitung können mittels einer Spritze Wirkstoffe an die Wundoberfläche unter der Folie appliziert werden. Nach der gewünschten Einwirkzeit können die Wirkstoffe gegebenenfalls zusammen mit dem Wundsekret über die Ableitung mittels einer Unterdruckquelle abgesaugt werden. Die Zuleitung weist ein selbsttätig schließendes Sicherheitsventil auf, welches durch die eingeführte Spritze zum Zuführen der flüssigen Wirkstoffe geöffnet wird. Ebenso ist an der Ableitung ein Absperrorgan vorgesehen, welches die Ableitung während der Einwirkungsdauer des Wirkstoffes verschließt.

Bei diesem bekannten Instillationsverband wird der Wirkstoff unmittelbar auf die Wundoberfläche gebracht und von der Wundoberfläche abgesaugt. Der Wirkstoff kann daher nur ungenau dosiert einwirken und insbesondere bei größeren Wundoberflächen ist eine gleichmäßige Einwirkung auf die gesamte Oberfläche kaum erreichbar. Eine Langzeit-Depotwirkung kann nicht realisiert werden.

Aus der US 4,382,441 ist es bekannt, zur Behandlung von Wundoberflächen eine Einlage aus einem porösen Material auf die Wundoberfläche aufzulegen und diese abdichtend zu überdekken. Der zu applizierende Wirkstoff wird kontinuierlich durch die Einlage geleitet, wozu eine Zuleitung und eine Ableitung in die Einlage führen. Die Kapillarwirkung der porösen Einlage begünstigt die Verteilung des zugeführten Wirkstoffes über die gesamte mit der Wundoberfläche in Berührung stehende Oberfläche der Einlage.

Die poröse Einlage ist weitgehend formstabil und auch die abdichtende Auflage ist vorzugsweise formstabil. Der flüssige Wirkstoff wird in kontinuierlicher Strömung durch die Einlage geleitet, wobei sich eine Strömungsverteilung ausbildet, bei welcher der flüssige Wirkstoff im wesentlichen in dem Bereich zwischen Zuleitung und Ableitung fließt, während die Randbereiche der Einlage kaum durchströmt werden. In diesen Randbereichen wird der Wirkstoff daher kaum ausgetauscht und auch in den Randbereichen aufgenommenes Wundsekret wird nur ungenügend abgeführt. Die relativ formstabile Einlage liegt zudem nicht in allen Bereichen gleichmäßig an der Wundcberfläche an, so daß auch hierdurch eine gleichmäßige Applikation des Wirkstoffes und eine gleichmäßige Abführung des Wundsekrets beeinträchtigt wird. Eine Depotwirkung ist nicht beabsichtigt.

Der Erfindung liegt die Aufgabe zugrunde, eine Vorrichtung zur Applikation von Wirkstoffen an einer Wundoberfläche zur Verfügung zu stellen, welche eine hohe Wirksamkeit des applizierten Wirkstoffes auf der gesamten Wundoberfläche gewährleistet, eine optimale Dosierung des Wirkstoffes ermöglicht und den Wundheilungsprozeß begünstigt.

Diese Aufgabe wird erfindungsgemäß gelöst durch eine Vorrichtung gemäß dem Anspruch 1.

Vorteilhafte Ausführungsformen und Weiterbildungen der Erfindung sind in den rückbezogenen Unteransprüchen angegeben.

Der Grundgedanke der Erfindung besteht darin, eine Einlage aus einem elastisch kompressiblen porösen Material auf die Wundoberfläche aufzulegen und die Wundoberfläche und die Einlage durch eine abdichtende Folie abzudecken, die die Wunde und die Einlage gegen die Atmosphäre abschließt. Die Zuleitung und die Ableitung sind jeweils mit steuerbaren Absperrorganen ausgestattet, die zeitlich so gesteuert werden, daß das Einleiten der Wirkstoffe und das Absaugen der Wirkstoffe und gegebenenfalls des Wundsekrets zeitlich voneinander getrennt sind. Zwischen dem Zeitintervall, in welchem das Absperrorgan der Zuleitung geöffnet ist und der Wirkstoff zugeführt wird, und dem Zeitpunkt, in welchem das Absperrorgan der Ableitung geöffnet wird, um den Wirkstoff und das Wundsekret abzusaugen, wird ein Einwirkungszeitintervall geschaltet, in welchem beide Absperrorgane geschlossen sind und der Wirkstoff statisch auf die Wundoberfläche einwirkt. Nach dem Absaugen des Wirkstoffes und des Wundsekrets wird außerdem ein Zeitintervall geschaltet, in welchem das Absperrorgan der Zuleitung geschlossen bleibt und ein Unterdruck im Bereich der Wunde aufrechterhalten wird. Hierzu kann das Absperrorgan der Ableitung geöffnet bleiben, um über die Unterdruckquelle einen konstanten Unterdruck im Wundbereich aufrechtzuerhalten und das Wundsekret abzusaugen. Das Absperrorgan der Ableitung kann auch geschlossen werden, so daß der anfangs erzeugte Unterdruck aufrechterhalten bleibt. Es ist auch möglich, in dieser Phase das Absperrorgan der Ableitung zeitweise gesteuert zu öffnen, um den Unterdruck wieder herzustellen, falls dieser unter einen vorgegebenen Wert nachläßt.

Das Einwirkungs-Zeitintervall, in welchem die Einlage mit dem Wirkstoff getränkt ist und der Wirkstoff mit Depotwirkung auf die Wundoberfläche appliziert wird, wird entsprechend der Art des Wirkstoffes, seiner Dosierung und der durch den Zustand der Wundoberfläche gegebenen Indikation gewählt. In den Unterdruckzeitintervallen, in welchen kein Wirkstoff appliziert wird und nur das Wundsekret abgesaugt wird, können die körpereigenen immunologischen Reparatur- und Abwehrprozesse des Gewebes ungestört ablaufen, so daß die Wundheilung optimiert wird. Es wechseln somit Einwirkungsphasen und Heilungsphasen zeitlich nacheinander ab. In den Einwirkungsphasen wird mittels Wirkstoffen, wie z.B. Antibiotika oder Antiseptika, aktiv in das Wundsystem eingegriffen, um Infektionen und dgl. zu bekämpfen. Da solche Wirkstoffe neben der gewünschten Hauptwirkung in der Regel auch unerwünschte Nebenwirkungen haben, werden im Anschluß an die Einwirkungsphase die Wirkstoffe abgesaugt und mit diesen das Wundsekret, welches die während der Einwirkungsphase entstehenden Zerfallsprodukte mit ihren unter Umständen toxischen Wirkungen enthält. In dem anschließenden Unterdruckzeitintervall kann der körpereigene immunologische Heilungsprozeß optimal ablaufen, ohne durch die unerwünschten Nebenwirkungen der Wirkstoffe und die bei der Bekämpfung der Wundinfektion entstehenden Zerfallsprodukte beeinträchtigt zu werden.

Vorzugsweise werden die Absperrorgane der Zuleitung und Ableitung zeitlich so gesteuert, daß die Einleitung der Wirkstoffe nur langsam und mit geringem Volumenstrom beginnt. Dadurch wird verhindert, daß durch ein zu plötzliches und schnelles Einleiten des Wirkstoffes Wundschmerzen verursacht oder verstärkt werden. Ebenso erfolgt das Öffnen des Absperrorgans der Ableitung zeitlich gesteuert in der Weise, daß der Unterdruck nur langsam zunmimmt. Ein zu schnell, schlagartig erzeugter Unterdruck führt ebenfalls zu erheblichen Wundschmerzen.

Die auf die Wundoberfläche aufgebrachte Einlage besteht aus einem elastisch kompressiblen porösen Material, vorzugsweise einem PVA-Schwamm (Polyvinylalkohol-Schwamm), wobei eine flexible Folie zur Abdeckung verwendet wird. Wird in dem durch die Folie abgedeckten Wundbereich ein Unterdruck erzeugt, so legt sich die Folie dicht auf die Wunde auf und drückt die Einlage zusammen. Dadurch legt sich die Einlage über ihre gesamte Fläche gleichmäßig dicht an die Wundoberfläche an. Dies begünstigt das Absaugen des Wundsekrets während des Unterdruckzeitintervalls. Wird zum Einleiten des Wirkstoffs die Zuleitung geöffnet, so saugt die poröse Einlage den Wirkstoff auf, wobei sie sich aufgrund ihrer elastischen Rückstellkraft ausdehnt. Dadurch wird bewirkt, daß die Einlage sich wie ein Schwamm gleichmäßig mit dem Wirkstoff vollsaugt. Der Wirkstoff ist über die gesamte Fläche der Einlage gleichmäßig verteilt und wirkt auf die gesamte Wundoberfläche in gleicher Weise ein. Begünstigt wird dies dadurch, daß sich die Einlage in der Unterdruckphase dicht an die Wundoberfläche anlegt. Die gleichmäßige Verteilung des Wirkstoffes über die gesamte Oberfläche wird während der Einwirkungszeit nicht beeinträchtigt, da nach dem Ende des Einleitens während des Einwirkungszeitintervalls ein statischer Zustand herrscht, in dem Zuleitung und Ableitung verschlossen sind.

Da sich die Wundbehandlung über eine längere Zeit, z. B. über einige Tage erstrecken kann, kann es vorkommen, daß insbesondere während einer längeren Unterdruckphase die Poren der Einlage geringfügig verkleben. Solche Verklebungen erhöhen den Widerstand für das Einleiten des Wirkstoffes. Dadurch kann ein Einleiten des Wirkstoffes unter Schwerkraft behindert werden. In solchen Fällen ist es zweckmäßig, zu Beginn des Einleitens des Wirkstoffes die poröse Einlage zunächst freizuspülen. Hierzu wird bei Beginn des Einleitens des Wirkstoffes zunächst ein kleineres Volumen des flüssigen Wirkstoffes unter Druck zugeführt, um die Poren der Einlage zu durchspülen und Verklebungen zu lösen. Das Einleiten unter Druck kann vorzugsweise durch eine Spritze erfolgen, die an die Zuleitung angeschlossen wird. Über den Kolbendruck der Spritze kann zunächst der Wirkstoff mit dosiertem Druck zum Freispülen zugeführt werden, bevor das Einleiten des Wirkstoffes durch Schwerkraft erfolgt. Gegebenenfalls ist es auch möglich, die erforderliche Menge des flüssigen Wirkstoffs während der gesamten Einleitungsphase mittels einer Spritze zuzuführen.

Im folgenden wird die Erfindung anhand von in der Zeichnung dargestellten Ausführungsbeispielen näher erläutert. Es zeigen
- Figur 1: die Vorrichtung zur Applikation von Wirkstoffen in einer ersten Ausführung,
- Figur 2: eine Abwandlung der Vorrichtung,
- Figur 3: ein Zeitdiagramm des Verfahrens, und
- Figuren 4 bis 8: verschiedene Ausführungsbeispiele für die Absperrorgane der Vorrichtung.

Zur Behandlung einer großflächigen tiefen Wunde 10 wird in diese eine Einlage 12 eingelegt. Die Einlage 12 besteht aus einem porösen elastisch kompressiblen Material, vorzugsweise aus einem offenporigen PVA-Schaumstoff. Die Einlage 12 wird entsprechend der Kontur der Wunde 10 zugeschnitten. Die Wunde 10 und die Einlage 12 werden durch eine Folie 14 abgedeckt und dichtend abgeschlossen. Die Folie 14 wird so zugeschnitten, daß sie die Einlage 12 und die Wunde 10 überdeckt und über die Ränder der Wunde hinausragt. Die Folie 14 wird rings um den Wundrand auf der Hautoberfläche dichtend befestigt, z.B. aufgeklebt. Die Folie 14 ist flexibel und besteht aus einem Kunststoffmaterial, welche die Diffusion von Wasserdampf gestattet, jedoch einen luftdichten Abschluß gewährleistet.

In die Einlage 12 wird ein Drainageschlauch 16, ein sogenannter Redon-Schlauch, eingezogen, der in seinem in der Einlage 12 liegenden Endbereich perforiert ist. Der nicht perforierte Bereich des Drainageschlauches 16 wird abgedichtet unter der Folie 14 herausgeführt.

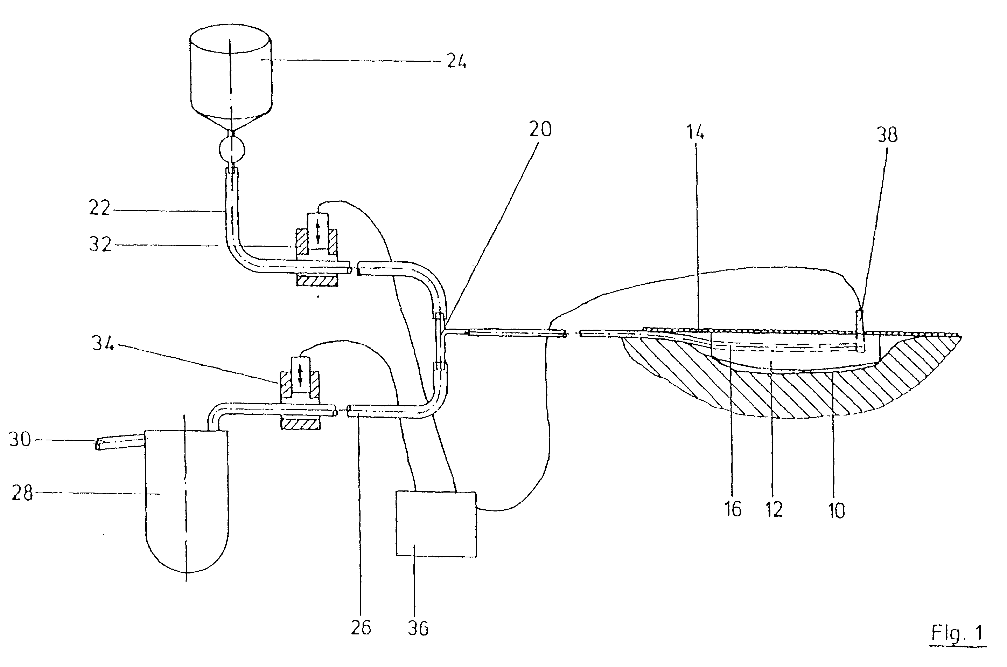

Im Ausführungsbeispiel der Figur 1 ist in das proximale Ende des Drainageschlauchs 16 ein T-förmiges Verzweigungsstück 20 eingesetzt. An den einen Anschluß des Verzweigungsstückes 20 ist ein Schlauch als Zuleitung 22 angesetzt, der zu einem an sich bekannten Infusionsbehälter 24 führt. An den anderen Anschluß des Verzweigungsstückes 20 ist ein Schlauch als Ableitung 26 angeschlossen, der zu einem Auffangbehälter 28 führt, an welchen über eine Anschlußleitung 30 eine Unterdruckquelle anschließbar ist. Ein solcher Auffangbehälter 28 ist ebenfalls an sich bekannt.

Der Zuleitung 22 ist ein Absperrorgan 32 zugeordnet und der Ableitung 26 ist ein Absperrorgan 34 zugeordnet. Die Absperrorgane 32 und 34 sind kontinuierlich zwischen einer Schließstellung und einer Offenstellung verstellbar und werden durch eine Steuerung 36 betätigt.

Im dargestellten Ausführungsbeispiel sind die Absperrorgane 32 und 34 jeweils als Schlauchklemmen ausgebildet, die eine Aufnahme aufweisen, in welche der Schlauch der Zuleitung 22 bzw. der Ableitung 26 eingelegt werden kann. Ein Stempel wird, wie in der Zeichnung durch Pfeile angedeutet ist, von der Steuerung 36 gesteuert elektromagnetisch, pneumatisch, hydraulisch oder in sonstiger bekannter Weise betätigt, um den eingelegten Schlauch gegen ein Widerlager zu drücken und quetschend zu verschließen oder um den Durchtrittsquerschnitt des Schlauches kontinuierlich freizugeben.

In den Wundraum unter der Folie 14 kann gegebenenfalls ein Drucksensor 38 eingesetzt werden, der den unter der Folie 14 herrschenden Druck mißt und der Steuerung 36 meldet.

In dem Infusionsbehälter 24 wird ein flüssiger Wirkstoff bevorratet, der bei geöffnetem Absperrorgan 32 und geschlossenem Absperrorgan 34 über die Zuleitung 22 und den Drainageschlauch 16 in die Einlage 12 und damit an die Wundoberfläche geleitet werden kann. Bei geschlossenem Absperrorgan 32 und geöffnetem Absperrorgan 34 können der Wirkstoff und das in der Wunde 10 erzeugte Wundsekret über die Ableitung 26 in den Auffangbehälter 28 abgesaugt werden.

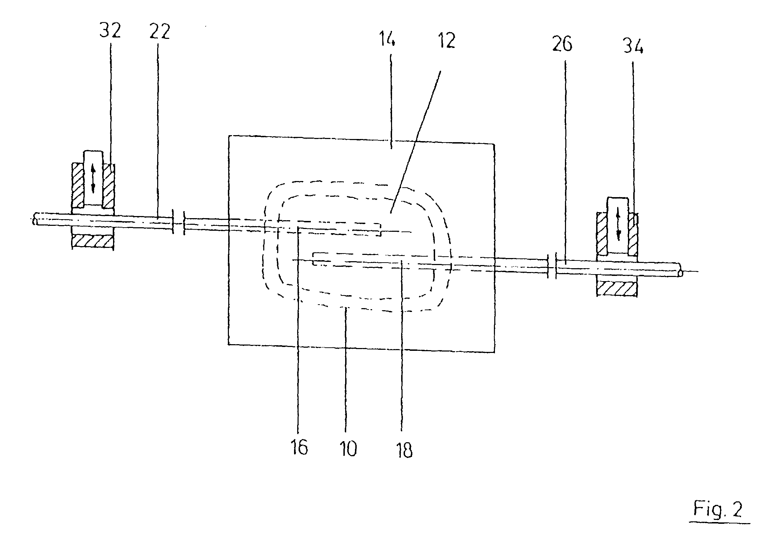

Figur 2 zeigt eine Abwandlung der Vorrichtung, die sich gegenüber dem Ausführungsbeispiel der Figur 1 darin unterscheidet, daß in die in die Wunde 10 eingelegte Einlage 12 zwei Drainageschläuche 16 und 18 eingezogen sind. Der Drainageschlauch 16 ist mit der Zuleitung 22 verbunden, während der Drainageschlauch 18 mit der Ableitung 26 verbunden ist. Eine Verzweigung entfällt dabei.

In dem Ausführungsbeispiel der Figur 1 bildet der Drainageschlauch 16 ein blindes Schlauchende, in welches einerseits der Wirkstoff eingeleitet und aus welchem andererseits der Wirkstoff abgesaugt wird. Dabei können beim Einleiten des flüssigen Wirkstoffes Gasbläschen in dem Drainageschlauch 16 gefangen werden, die das Einleiten des Wirkstoffes behindern. Diese Schwierigkeiten können bei der Ausführung der Figur 2 nicht auftreten, da eventuell in dem Drainageschlauch 16 gefangene Gasbläschen über den Drainageschlauch 18 abgesaugt werden. Die Ausführung der Figur 1 hat jedoch den Vorteil, daß nur ein Drainageschlauch abgedichtet unter die Folie 14 geführt werden muß.

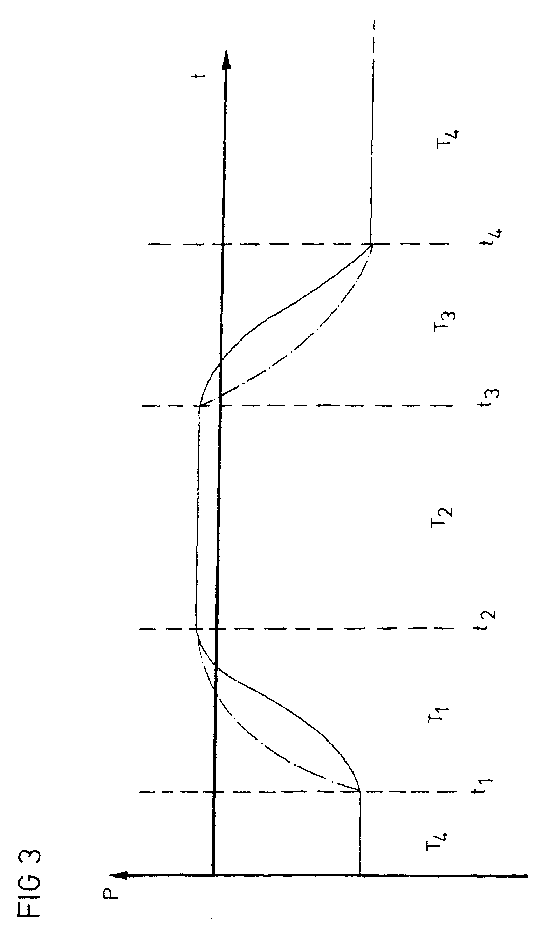

Anhand der Figur 3 wird das mit der Vorrichtung der Figuren 1 und 2 durchgeführte erfindungsgemäße Verfahren erläutert.

In Figur 3 ist der Druck P in der Wunde 10 unter der Folie 14 als Funktion der Zeit t dargestellt. Die eingezeichnete Abszissenachse entspricht dabei dem Atmosphärendruck.

Zum Zeitpunkt t₁ sind die Absperrorgane 32 und 34 gesteuert durch die Steuerung 36 geschlossen. In der Wunde herrscht ein Unterdruck von etwa 10 bis 80 kPa. Aufgrund dieses Unterdrucks wird die Folie 14 gegen die Wundoberfläche gedrückt, wobei die elastische Einlage 12 komprimiert wird. Zum Zeitpunkt t₁ wird nun gesteuert durch die Steuerung 36 das Absperrorgan 32 geöffnet, so daß der flüssige Wirkstoff aus dem Infusionsbehälter 24 über die Zuleitung 22 und den Drainageschlauch 16 in die Einlage 12 fließen kann. Während des Einleit-Zeitintervalls T₁ saugt sich die Einlage 12 mit flüssigem Wirkstoff voll, wobei sie sich aufgrund ihrer elastischen Rückstellkraft ausdehnt. Zum Zeitpunkt t₂ ist die Einlage 12 mit dem flüssigen Wirkstoff vollgesogen, wobei unter der Folie 14 ein gewisser Überdruck herrsche, der vorzugsweise durch die Höhe des Infusionsbehälters 24 gegenüber der Wunde 10 bestimmt ist. Gegebenenfalls kann auch eine mittels des Drucksensors 38 druckgesteuerte Pumpe in die Zuleitung 22 eingeschaltet werden.

Das Öffnen des Absperrorgans 32 der Zuleitung 22 in dem Einleit-Zeitintervall T₁ erfolgt mittels der Steuerung 36 zeitlich in der Weise gesteuert, daß der durchtretende Volumenstrom des flüssigen Wirkstoffes nur langsam ansteigt, wie dies in Figur 3 durch die ausgezogene Linie dargestellt ist. Ein schlagartiges Öffnen des Absperrorgans 32 würde zu einem sehr schnellen Einfließen des Wirkstoffes führen, wie es in Figur 3 strichpunktiert eingezeichnet ist. Dies könnte zu Wundschmerzen des Patienten führen, insbesondere da der flüssige Wirkstoff in der Regel nicht die Körpertemperatur des Patienten aufweist.

Während der Unterdruckphase können sich unter Umständen die Poren der komprimierten Einlage 12 verkleben. Ein solches Verkleben behindert das Einfließen des Wirkstoffes allein durch die Schwerkraft, die sich aus der Höhe des Infusionsbehälters 24 gegenüber der Wunde 10 ergibt. Solche eventuellen Verklebungen der Poren können freigespült werden, indem beim Öffnen des Absperrorgans 32 zunächst ein gewisses Volumen des flüssigen Wirkstoffs unter Druck eingeleitet wird. Hierzu kann ein entsprechendes Volumen des Wirkstoffes mittels einer Kolbenspritze über die Zuleitung 22 eingeleitet werden. Die Kolbenspritze wird dabei zweckmäßigerweise an die Zuleitung 22 angeschlossen, wozu beispielsweise ein Dreiwegehahn in die Zuleitung 22 eingesetzt werden kann, an welchen die Kolbenspritze angeschlossen ist.

Sobald sich die Einlage 12 mit dem flüssigen Wirkstoff vollgesogen hat, wird zum Zeitpunkt t₂ das Absperrorgan 32 der Zuleitung 22 geschlossen. Für ein Einwirkungs-Zeitintervall T₂ bleiben nun die Absperrorgane 32 und 34 der Zuleitung 22 und der Ableitung 26 geschlossen, so daß der in der Einlage 12 enthaltene Wirkstoff auf die Oberfläche der Wunde 10 einwirken kann. Die Dauer des Einwirkungs-Zeitintervalls T₂ kann der Steuerung 36 vorgegeben werden und bestimmt sich nach der Art und dem Zustand der Wunde 10 und nach Art und Konzentration des Wirkstoffes. Wenn der Wirkstoff in dem Zeitintervall T₂ ausreichend auf die Wundoberfläche eingewirkt hat, wird zum Zeitpunkt t₃ das Absperrorgan 34 der Ableitung 26 geöffnet. Dadurch wird über den über die Anschlußleitung 30 anstehenden Unterdruck der flüssige Wirkstoff über den Drainageschlauch 16 (in Figur 1 ) bzw. den Drainageschlauch 18 (in Figur 2) aus der Einlage 12 und der Wunde 10 abgesaugt. Gleichzeitig wird die Wundflüssigkeit abgesaugt, die sich in dem Einwirkungs-Zeitintervall T₂ in der Wunde 10 angesammelt hat und durch die Einwirkung des Wirkstoffes erzeugte Zerfalls- und Zersetzungsprodukte enthält.

Das Öffnen des Absperrorgans 34 wird mittels der Steuerung 36 zeitlich so gesteuert, daß sich der Durchtrittsquerschnitt der Ableitung 26 nur langsam öffnet und der Unterdruck in der Einlage 12 und der Wunde 10 nur langsam zunimmt, wie dies in Figur 3 durch die ausgezogene Linie gezeigt ist. Ein sofortiges vollständiges Öffnen des Absperrorgans 34 würde zu einem sehr steilen Druckabfall im Bereich der Wunde führen, wie dies in Figur 3 strichpunktiert gezeichnet ist, was mit Wundschmerzen für den Patienten verbunden wäre.

Ist zum Zeitpunkt t₄ der ursprüngliche Unterdruck wieder erreicht, was gegebenenfalls durch den Drucksensor 38 überwacht werden kann, so ist der flüssige Wirkstoff wieder vollständig aus der Wunde 10 und der Einlage 12 entfernt. Der Unterdruck wird nun über ein Unterdruck-Zeitintervall T₄ aufrechterhalten. Dabei wird in der Regel das Absperrorgan 34 geöffnet bleiben, so daß der Unterdruck kontinuierlich durch die Unterdruckquelle 30 aufrechterhalten wird und das entstehende Wundsekret in den Auffangbehälter 28 kontinuierlich abgesaugt wird. Es ist auch möglich, das Absperrorgan 34 zu schließen oder zeitweise zu schließen und nur kurzzeitig zu öffnen, wenn der durch den Drucksensor 38 überwachte Unterdruck regeneriert werden muß.

Soll die nächste Behandlung der Wunde 10 mit einem flüssigen Wirkstoff erfolgen, so wird zum Zeitpunkt t₁ das Absperrorgan 34 geschlossen und das Absperrorgan 32 der Zuleitung 22 wieder geöffnet, so daß der beschriebene Zyklus wieder von vorn beginnt.

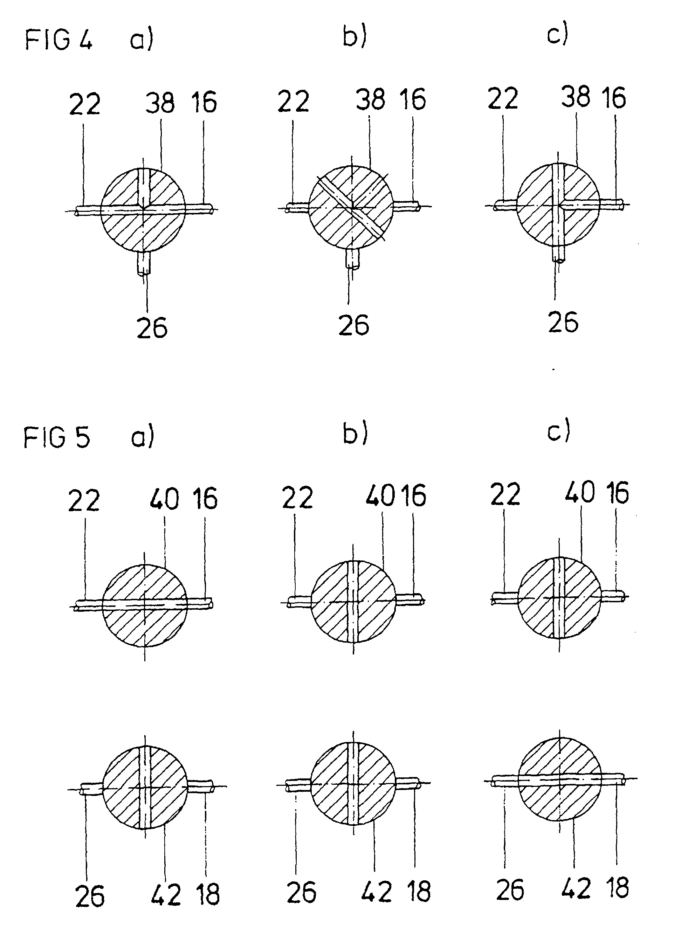

Anstelle der in den Figuren 1 und 2 gezeigten elektromagnetisch, pneumatisch oder hydraulisch betätigten Schlauchklemmen 32 und 34 können die Absperrorgane für die Zuleitung 22 und die Ableitung 26 auch als Mehrwegehähne ausgebildet sein. Entsprechende Ausführungsbeispiele sind in den Figuren 4 bis 8 gezeigt. Die Ausbildung der Absperrorgane als Mehrwegehähne ergibt einen einfachen Aufbau und ein zuverlässiges Schalten der Absperrorgane. Insbesondere ergibt sich eine einfache Steuerung der Absperrorgane, wenn die Mehrwegehähne mittels elektrischer Schrittmotoren geschaltet werden, was in einfacher Weise durch Antrieb der Hahnküken über die jeweiligen Schrittmotoren erfolgt. Das Schalten der Mehrwegehähne über Schrittmotore erlaubt eine einfache elektronische Steuerung. Diese Steuerung kann in einfacher Weise und mit großer Flexibilität programmiert werden, um eine Anpassung an den gewünschten Behandlungszyklus zu erreichen. Die Steuerung und der Behandlungszyklus können auf diese Weise individuell für jeden Patienten und jede Indikation programmiert werden. Auch das Öffnen und Schließen der Mehrwegehähne kann mittels der Schrittmotore elektronisch genau und flexibel zeitlich gesteuert werden, um den in Figur 3 gezeigten Druckverlauf zu realisieren.

In den Figuren 4 bis 8 sind jeweils nur die Absperrorgane der Vorrichtung gezeigt. Im übrigen entspricht die Vorrichtung den Ausführungsbeispielen der Figuren 1 und 2.

In dem Ausführungsbeispiel der Figur 4 ist nur ein Drainageschlauch 16 vorgesehen. Die Zuleitung 22 und die Ableitung 26 sind alternativ über einen Dreiwegehahn 38 mit dem Drainageschlauch 16 verbindbar. Mittels der elektronischen Steuerung 36 wird programmierbar ein nicht dargestellter Schrittmotor gesteuert, der den Dreiwegehahn 38 betätigt. In der Stellung a ist die Zuleitung 22 über den Dreiwegehahn 38 an den Drainageschlauch 16 angeschlossen, so daß der Wirkstoff in die poröse Einlage 12 eingeleitet werden kann. Anschließend wird der Dreiwegehahn 38 in die Stellung b gedreht, in welcher der Drainageschlauch 16 gesperrt ist, um den eingeleiteten Wirkstoff auf die Wunde einwirken zu lassen. Zum Absaugen wird der Dreiwegehahn 38 in die Stellung c gedreht, in welcher der Drainageschlauch 16 an die Ableitung 26 angeschlossen ist.

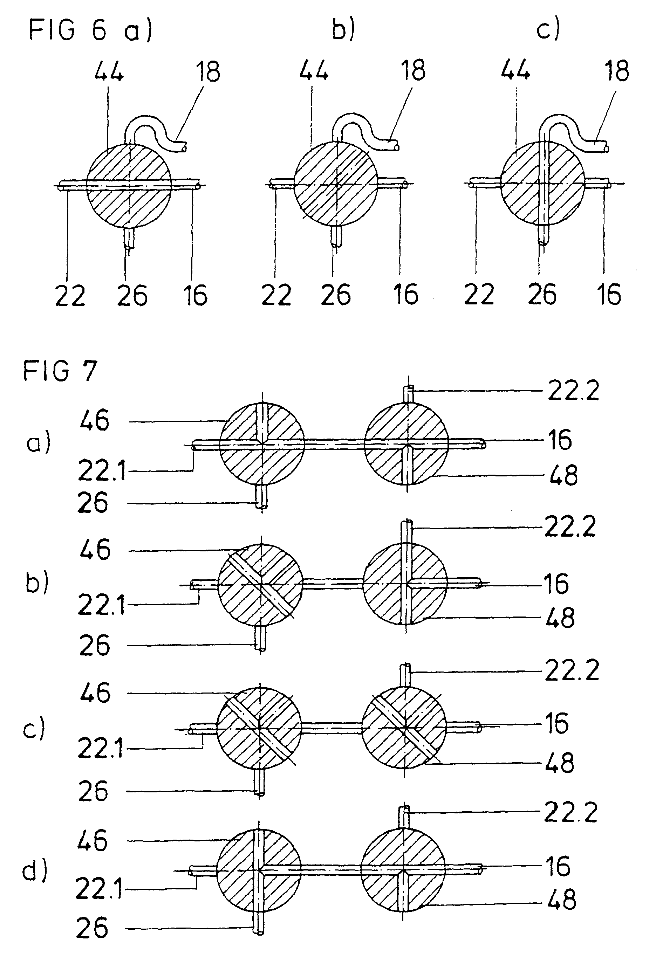

Das Ausführungsbeispiel der Figur 5 zeigt die Vorrichtung mit einem Drainageschlauch 16 für die Zuleitung und einem Drainageschlauch 18 für das Absaugen. Sowohl in die Zuleitung 22 als auch in die Ableitung 26 ist jeweils ein Zweiwegehahn 40 bzw. 42 eingeschaltet, so daß sich die Funktionsweise ergibt, wie sie in dem Ausführungsbeispiel der Figur 2 beschrieben ist. In der Stellung a ist der Zweiwegehahn 40 geöffnet und der Zweiwegehahn 42 geschlossen, so daß die Zuleitung 22 an den Drainageschlauch 16 angeschlossen ist, während der Drainageschlauch 18 gesperrt ist. In dieser Stellung a wird der flüssige Wirkstoff über den Drainageschlauch 16 in die Einlage 12 eingeleitet. Anschließend wird der Zweiwegehahn 40 gesperrt, so daß die Stellung b eingenommen wird. Sowohl die Zuleitung 22 als auch die Ableitung 26 sind abgesperrt, so daß der von der Einlage 12 aufgenommene Wirkstoff einwirken kann. Nach Beendigung der Einwirkungsphase wird der Zweiwegehahn 42 geöffnet, entsprechend der Stellung c, so daß nun der Wirkstoff und eventuelles Sekret über den Drainageschlauch 18 und die Ableitung 26 abgesaugt werden können. Nach einem mehr oder weniger langen Unterdruck-Intervall wird erneut in die Stellung a umgeschaltet, um wiederum einen Behandlungswirkstoff zuzuführen.

Die Ausführung der Figur 6 entspricht funktionsmäßig dem Ausführungsbeispiel der Figur 5. Es sind lediglich anstelle der zwei Zweiwegehähne 40 und 42 ein Vierwegehahn 44 vorgesehen. Der Vierwegehahn 44 verbindet in Stellung a die Zuleitung 22 mit dem Drainageschlauch 16, sperrt in Stellung b sowohl die Zuleitung 22 als auch die Ableitung 26 und verbindet in Stellung c die Ableitung 26 mit dem Drainageschlauch 18.

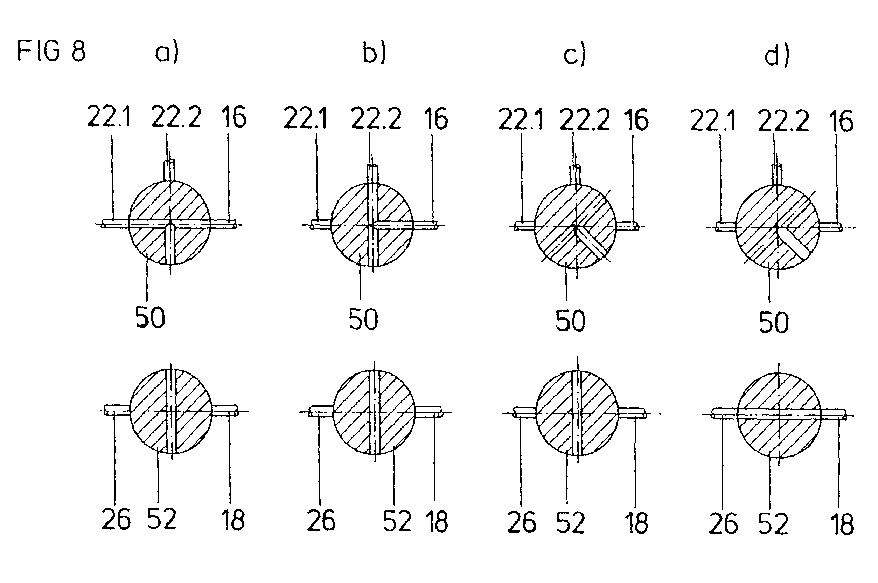

Bei den bisher beschriebenen Ausführungen ist jeweils nur eine Zuleitung 22 vorgesehen, so daß nur ein Infusionsbehälter 24 angeschlossen werden kann. Sollen unterschiedliche Wirkstoffe zugeführt werden, so muß der mit der Zuleitung 22 konnektierte Infusionsbehälter 24 ausgewechselt werden. Ebenso muß der Infusionsbehälter 24 entfernt und gegen eine Kolbenspritze ausgetauscht werden, wenn die Einlage 12 im Falle von Verklebungen der Poren freigespült werden soll. Diese Nachteile können durch die Ausführungsbeispiele der Figuren 7 und 8 behoben werden.

Das Ausführungsbeispiel der Figur 7 entspricht insoweit dem Ausführungsbeispiel der Figur 1, als nur ein Drainageschlauch 16 für die Zuleitung und die Ableitung vorgesehen ist. Der Drainageschlauch 16 ist über zwei in Reihe angeordnete Dreiwegehähne 46 und 48 angeschlossen. Der erste Dreiwegehahn 46 schließt wahlweise eine erste Zuleitung 22.1 oder die Ableitung 26 an eine Verbindungsleitung zu dem zweiten Dreiwegehahn 48. Der zweite Dreiwegehahn 48 schließt wahlweise diese Verbindungsleitung oder eine zweite Zuleitung 22.2 an den Drainageschlauch 16 an. In der Stellung a verbindet der erste Dreiwegehahn 46 die erste Zuleitung 22.1 mit der Verbindungsleitung und der zweite Dreiwegehahn 48 diese Verbindungsleitung mit dem Drainageschlauch 16. Es kann über die erste Zuleitung 22.1 ein erster Wirkstoff eingeleitet werden. Die zweite Zuleitung 22.2 und die Ableitung 26 sind gesperrt. In Stellung b sperrt der erste Dreiwegehahn 46 sämtliche Anschlüsse, während der zweite Dreiwegehahn 48 die zweite Zuleitung 22.2 mit dem Drainageschlauch 16 verbindet. In dieser Stellung kann über die Zuleitung 22.2 ein zweiter Wirkstoff eingeleitet werden. In der Stellung c sperren beide Dreiwegehähne 46 und 48 sämtliche Zuleitungen, so daß die eingeleiteten Wirkstoffe über ein steuerbares Zeitintervall einwirken können. Anschließend wird in der Stellung d der Drainageschlauch 16 über den zweiten Dreiwegehahn 48 mit der Verbindungsleitung verbunden, während der erste Dreiwegehahn 46 die Verbindungsleitung mit der Ableitung 26 verbindet. Es können nun die Wirkstoffe und ein eventuelles Wundsekret aus der Einlage 12 über den Drainageschlauch 16 und die Ableitung 26 abgesaugt werden.

Die Ausführung der Figur 7 eignet sich auch dazu, zu Beginn der Einleitungsphase die Einlage 12 freizuspülen. In diesem Falle wird an die Zuleitung 22.1 eine Kolbenspritze angeschlossen, während an die Zuleitung 22.2 der Infusionsbehälter 24 angeschlossen wird. Zunächst wird in der Stellung a mittels der Kolbenspritze, die verklebte Einlage 12 freigespült, um dann in der Stellung b den Wirkstoff aus den Infusionsbehälter 22 durch Schwerkraft über die zweite Zuleitung 22.2 zuzuführen. Das Freispülen mittels einer Kolbenspritze kann somit durchgeführt werden, ohne daß die Konnektierung der Anschlüsse geändert werden muß.

In dieser Ausführung ist es auch möglich, den an der zweiten Zuleitung 22.2 angeschlossenen Infusionsbehälter 24 nur als Vorratsbehälter für den flüssigen Wirkstoff zu verwenden und diesen ausschließlich mittels der an der ersten Zuleitung 22.1 angeschlossenen Spritze zu applizieren. Hierzu wird der erste Dreiwegehahn 46 in die Stellung der Figur 7a gebracht, während der zweite Dreiwegehahn 48 gegenüber dieser Stellung so verdreht wird, daß er die zweite Zuleitung 22.2 mit der Verbindungsleitung zu dem ersten Dreiwegehahn 46 verbindet. Es kann nun der flüssige Wirkstoff aus dem an die Zuleitung 22.2 angeschlossenen Infusionsbehälter 24 über den ersten Dreiwegehahn 46 in die an der ersten Zuleitung 22.1 angeschlossene Kolbenspritze geleitet werden, um diese zu füllen. Sobald die Kolbenspritze gefüllt ist, wird der zweite Dreiwegehahn 48 in die Stellung der Figur 7a gebracht, so daß der flüssige Wirkstoff nun mittels der Kolbenspritze appliziert werden kann.

Figur 8 zeigt eine Ausführung, die wiederum im Prinzip der Ausführung der Figur 2 entspricht, bei welcher ein Drainageschlauch 16 für die Zuleitung und ein Drainageschlauch 18 für die Ableitung vorgesehen sind. Die Einleitung kann jedoch über zwei Zuleitungen 22.1 und 22.2 erfolgen. Es besteht somit auch hier die Möglichkeit über die Zuleitungen 22.1 und 22.2 zwei verschiedene Wirkstoffe zuzuführen oder über eine der Zuleitungen den Wirkstoff über eine Spritze und über die andere Zuleitung den Wirkstoff aus einem Infusionsbehälter zuzuleiten. Der Drainageschlauch 16 ist über einen Dreiwegehahn 50 mit den beiden Zuleitungen 22.1 und 22.2 verbindbar, während der Drainageschlauch 18 über einen Zweiwegehahn 52 an die Ableitung 26 angeschlossen ist. In der Stellung a ist die erste Zuleitung 22.1 mit dem Drainageschlauch 16 verbunden, um einen ersten Wirkstoff zuzuleiten oder um die Einlage 12 freizuspülen. Der Zweiwegehahn 52 sperrt den zweiten Drainageschlauch 18. In der Stellung b wird ein zweiter Wirkstoff über die zweite Zuleitung 22.2 zugeleitet. In der Stellung c sind sowohl der Dreiwegehahn 50 als auch der Zweiwegehahn 52 für das Einwirken des Wirkstoffes gesperrt. In der Stellung d sperrt der Dreiwegehahn 50 sämtliche Anschlüsse, während der Zweiwegehahn 52 den Drainageschlauch 18 mit der Ableitung 26 verbindet, um die Wirkstoffe abzusaugen.

Auch hier kann an den Anschluß 22.2 ein Infusionsbehälter 24 angeschlossen werden, der nur als Vorratsbehälter für den flüssigen Wirkstoff verwendet wird, während dieser flüssige Wirkstoff mittels einer an die Zuleitung 22.1 angeschlossenen Spritze appliziert wird. In einer zu der Stellung b um 180° gedrehten Stellung verbindet dabei der Dreiwegehahn 50 den an die Zuleitung 22.2 angeschlossenen Vorrats-Infusionsbehälter 24 mit der an die Zuleitung 22.1 angeschlossenen Kolbenspritze, um diese zu füllen.

## Patentansprüche

1. Vorrichtung zur Applikation von Wirkstoffen an einer Wundoberfläche, mit einer Einlage aus einem porösen Material zum Auflegen auf die Wundoberfläche, mit einer abdichtenden Auflage zum Überdekken der Wundoberfläche und der Einlage, die abdichtend an der Hautoberfläche befestigbar ist, mit wenigstens einer in die Einlage führenden Zuleitung für einen flüssigen Wirkstoff und mit wenigstens einer in die Einlage führenden Ableitung, die an eine Unterdruckquelle anschließbar ist, **dadurch gekennzeichnet, daß** die Zuleitung (22; 22.1, 22.2) ein steuerbares Absperrorgan (32; 38; 40; 44; 46; 48; 50) aufweist, daß die Ableitung (26) ein steuerbares Absperrorgan (34; 38; 42; 44; 46; 48; 52) aufweist und daß eine Steuerung (36) vorgesehen ist, die diese Absperrorgane (32; 34; 38; 40; 42; 44; 46; 48; 50; 52) zeitlich so steuert, daß das Absperrorgan (32; 34; 38; 40; 42; 44; 46; 48; 50; 52) der Zuleitung (22; 22.1, 22.2) und das Absperrorgan (34; 38; 40; 42; 44; 46; 48; 52) der Ableitung (26) nicht gleichzeitig sich überlappend geöffnet sind und daß zwischen dem Schließen des Absperrorgans (32; 34; 38; 40; 42; 44; 46; 48; 50; 52) der Zuleitung (22; 22.1, 22.2) und dem Öffnen des Absperrorgans (34; 38; 40; 42; 44; 46; 48; 50; 52) der Ableitung (26) ein Einwirkungs-Zeitintervall (T₂) geschaltet ist.

2. Vorrichtung nach Anspruch 1, **dadurch gekennzeichnet, daß** die Einlage (12) aus einem elastisch kompressiblen porösen Material besteht.

3. Vorrichtung nach Anspruch 2, **dadurch gekennzeichnet, daß** die Einlage (12) aus einem offenporigen PVA-Schaumstoff besteht.

4. Vorrichtung nach einem der Ansprüche 1 bis 3, **dadurch gekennzeichnet, daß** die abdichtende Auflage eine flexible Folie (14) ist, die luftdicht ist, jedoch die Diffusion von Wasserdampf zuläßt.

5. Vorrichtung nach einem der Ansprüche 1 bis 4, **dadurch gekennzeichnet, daß** die Zuleitung (22; 22.1, 22.2) und die Ableitung (26) über einen gemeinsamen Drainageschlauch (16) in die Einlage (12) führen.

6. Vorrichtung nach einem der Ansprüche 1 bis 5, **dadurch gekennzeichnet, daß** die Zuleitung (22; 22.1, 22.2) und die Ableitung (26) jeweils über gesonderte Drainageschläuche (16 bzw. 18) in die Einlage (12) führen.

7. Vorrichtung nach Anspruch 1, **dadurch gekennzeichnet, daß** der Öffnungsvorgang des Absperrorgans (32; 34; 38; 40; 42; 44; 46; 48; 50; 52) der Zuleitung (22; 22.1, 22.2) mittels der Steuerung (36) zeitlich steuerbar ist.

8. Vorrichtung nach Anspruch 1, **dadurch gekennzeichnet, daß** der Öffnungsvorgang des Absperrorgans (34; 38; 40; 42; 44; 46; 48; 50; 52) der Ableitung (26) mittels der Steuerung (36) zeitlich steuerbar ist.

9. Vorrichtung nach Anspruch 1, **dadurch gekennzeichnet, daß** die Steuerung (36) nach dem Absaugen (T₃) ein Unterdruck- Zeitintervall (T₄) bestimmt, in welchem ein vorgegebener Unterdruck in der Einlage (12) aufrechterhalten wird.

10. Vorrichtung nach einem der Ansprüche 1 bis 9, **dadurch gekennzeichnet, daß** ein Drucksensor (38) unter die abdichtende Auflage (14) einsetzbar ist, der mit der Steuerung (36) wirkungsmäßig verbunden ist.

11. Vorrichtung nach einem der Ansprüche 1 bis 10, **dadurch gekennzeichnet, daß** die Absperrorgane (32, 34) elektromagnetisch, pneumatisch oder hydraulisch gesteuerte Schlauchklemmen sind.

12. Vorrichtung nach Anspruch 11, **dadurch gekennzeichnet, daß** die als Schlauch ausgebildete Zuleitung (22) bzw. Ableitung (34) in eine Aufnahme der Schlauchklemme (32) bzw. (34) einlegbar ist und durch einen gesteuert betätigten Stempel gegen ein Widerlager quetschbar ist.

13. Vorrichtung nach einem der Ansprüche 1 bis 10, **dadurch gekennzeichnet, daß** die Absperrorgane Mehrwegehähne (38; 40; 42; 44; 46; 48; 50; 52) sind.

14. Vorrichtung nach Anspruch 13, **dadurch gekennzeichnet, daß** die Mehrweghähne (38; 40; 42; 44; 46; 48; 50; 52) mittels durch die Steuerung (36) gesteuerter Schrittmotoren betätigbar sind.

15. Vorrichtung nach Anspruch 14, **dadurch gekennzeichnet, daß** die Steuerung (36) eine programmierbare elektronische Steuerung ist.

## Claims

1. Device for the application of active agents to a wound surface with an insert comprising a porous material for application on the surface of the wound with a sealing layer to cover the wound surface and the insert, which may be attached in a sealing manner to the surface of the skin with at least one supply line for a liquid active agent running into the insert and with at least one drainage line which may be connected to an underpressure source running into the insert, **characterised in that** the supply line (22, 22.1, 22,2) has a controllable shut-off organ (32, 38, 40, 44, 46, 48, 50), the drainage line (26) has an controllable shut-off organ (34, 38, 42, 44, 46. 48, 52) and that a control (36) is provided for the time-control of these shut-off organs (32, 34, 38, 40, 42, 44, 46, 48, 50, 52) in such a way that the shut-off organ (32, 34, 38, 40, 42, 44, 46, 48, 50, 52) for the supply line (22, 22.1, 22.2) and the shut-off organ (34, 38, 40, 42, 44, 46, 48, 52) for the drainage line (26) are not simultaneously and overlappingly open and that there is an exposure time interval (T₂) between the closing of the shut-off organ (32, 34, 38, 40, 42, 44, 46, 48, 50, 52) for the supply line (22, 22.1, 22.2) and the opening of the shut-off organ (34, 38, 40, 42, 44, 46, 48, 50, 52), for the drainage line (26).

2. Device according to Claim 1, **characterised in that** the insert (12) comprises an elastically compressible, porous material.

3. Device according to Claim 2, **characterised in that** the insert (12) comprises an open-cell PVA foam

4. Device according to any one of claims 1 to 3, **characterised in that** the sealing layer is a flexible film (14) which is airtight, but permits the diffusion of water vapour.

5. Device according to any one of claims 1 to 4, **characterised in that** the supply line (22, 22.1, 22.2) and the drainage line (26) run into the insert via a common drainage hose.

6. Device according to any one of claims 1 to 5, **characterised in that** the supply line (22, 22.1, 22.2) and the drainage line (26) each lead into the insert (12) via separate drainage hoses (16 or 18).

7. Device according to claim 1, **characterised in that** the opening process for the shut-off organ (32, 34, 40, 42, 44, 46, 48, 50, 52) for the supply line (22, 22.1, 22.2) may be time-controlled by means of the control (36).

8. Device according to claim 1, **characterised in that** the opening process for the shut-off organ (34, 38, 40, 42, 44, 46, 48, 50, 52) for the drainage line (26) may be time-controlled by means of the control (36).

9. Device according to claim 2, **characterised in that**, after the removal by suction, the control (36) determines an underpressure time interval (T₄) in which a specified underpressure is maintained in the insert (12).

10. Device according to any one of claims 1 to 9, **characterised in that** a pressure sensor (38) functionally connected to the control (36) may be used under the sealing layer (14).

11. Device according to any one of claims 1 to 10, **characterised in that** the shut-off organs (32, 34) are electromagnetically, pneumatically or hydraulically controlled hose clamps.

12. Device according to claim 11, **characterised in that** the supply line (22) or drainage line (34) embodied as a hose may be inserted into a holding fixture for the hose clamp (32) or (34) and may be squeezed against an abutment by a controllably actuated punch.

13. Device according to any one of claims 1 to 10, **characterised in that** the shut-off organs are multi-way cocks (38, 40, 42, 44, 46. 48. 50. 52).

14. Device according to claim 13 **characterised in that** the multi-way cocks (38, 40, 42, 44, 46, 48, 50, 52) may be actuated by the stepping motors controlled by the control (36).

15. Device according to claim 14, **characterised in that** the control (36) is a programmable electronic control.

## Revendications

1. Dispositif pour l'administration de principes actifs à la surface d'une plaie, avec un support composé d'un matériel poreux destiné à être appliqué sur la surface de la plaie, avec une couche d'étanchéité pour recouvrir la surface de la plaie et le support, pouvant être fixée de manière étanche sur la surface de la peau, avec au moins une arrivée pénétrant dans le support pour un principe actif liquide et avec au moins une évacuation pénétrant dans le support, qui peut être reliée à une source de dépression,
**caractérisé en ce que**
l'arrivée (22 ; 22.1 ; 22.2) est munie d'un dispositif d'obturation pouvant être commandé (32 ; 38 ; 40 ; 44 ; 46 ; 48 ; 50), l'évacuation (26) est munie d'un dispositif d'obturation pouvant être commandé (34 ; 38 ; 42 ; 44 ; 46 ; 48 ; 52) et une commande (36) est prévue pour commander chronologiquement ces dispositifs d'obturation (32 ; 34 ; 38 ; 40 ; 42 ; 44 ; 46 ; 48 ; 50 ; 52) de telle sorte que le dispositif d'obturation (32 ; 34 ; 38 ; 40 ; 42 ; 44 ; 46 ; 48 ; 50 ; 52) de l'arrivée (22 ; 22.1 ; 22.2) et le dispositif d'obturation (34 ; 38 ; 40 ; 42 ; 44 ; 46 ; 48 ; 52) de l'évacuation (26) s'ouvrent de manière non simultanée en se chevauchant et qu'entre la fermeture du dispositif d'obturation (32 ; 34 ; 38 ; 40 ; 42 ; 44 ; 46 ; 48 ; 50 ; 52) de l'arrivée (22 ; 22.1 ; 22.2) et l'ouverture du dispositif d'obturation (34 ; 38 ; 40 ; 42 ; 44 ; 46 ; 48 ; 50 ; 52) de l'évacuation (26) un intervalle de temps d'action (T₂) s'écoule.

2. Dispositif selon la revendication 1,
**caractérisé en ce que**
le support (12) est composé d'un matériel poreux, compressible et élastique.

3. Dispositif selon la revendication 2,
**caractérisé en ce que**
le support (12) est composé d'une mousse de PVA à pores ouverts.

4. Dispositif selon l'une quelconque des revendications 1 à 3,
**caractérisé en ce que**
la couche d'étanchéité est un film flexible (14) qui est imperméable à l'air, mais permet la diffusion de la vapeur d'eau.

5. Dispositif selon l'une quelconque des revendications 1 à 4,
**caractérisé en ce que**
l'arrivée (22 ; 22.1 ; 22.2) et l'évacuation (26) conduisent par l'intermédiaire d'un tube de drainage commun (16) dans le support (12).

6. Dispositif selon l'une quelconque des revendications 1 à 5,
**caractérisé en ce que**
l'arrivée (22 ; 22.1 ; 22.2) et l'évacuation (26) conduisent chacune par l'intermédiaire d'un tube de drainage particulier (16 ou 18) dans le support (12).

7. Dispositif selon la revendication 1,
**caractérisé en ce que**
le procédé d'ouverture du dispositif d'obturation (32 ; 34 ; 38 ; 40 ; 42 ; 44 ; 46 ; 48 ; 50 ; 52) de l'arrivée (22 ; 22.1 ; 22.2) est contrôlé chronologiquement à l'aide d'une commande (36).

8. Dispositif selon la revendication 1,
**caractérisé en ce que**
le procédé d'ouverture du dispositif d'obturation (34 ; 38 ; 40 ; 42 ; 44 ; 46 ; 48 ; 50 ; 52) de l'évacuation (26) est contrôlé chronologiquement à l'aide d'une commande (36).

9. Dispositif selon la revendication 1,
**caractérisé en ce que**
la commande (36) après l'absorption (T₃) fixe un intervalle de temps de dépression (T₄) au cours duquel une dépression réglée par avance est maintenue dans le support (12).

10. Dispositif selon l'une quelconque des revendications 1 à 9,
**caractérisé en ce qu'**
un capteur de pression (38) peut être inséré sous la couche d'étanchéité (14), qui est relié pour son mode de fonctionnement à la commande (36).

11. Dispositif selon l'une quelconque des revendications 1 à 10,
**caractérisé en ce que**
les dispositifs d'obturation (32, 34) sont des colliers de serrage contrôlés par un dispositif électromagnétique, pneumatique ou hydraulique.

12. Dispositif selon la revendication 11,
**caractérisé en ce que**
l'arrivée (22) ou l'évacuation (26) constituée d'un tube peut être insérée dans le logement d'un collier de serrage (32) ou (34), et peut être comprimée par un piston commandé contre une butée.

13. Dispositif selon l'une quelconque des revendications 1 à 10,
**caractérisé en ce que**
les dispositifs d'obturation (38 ; 40 ; 42 ; 44 ; 46 ; 48 ; 50 ; 52) sont des robinets à voies multiples.

14. Dispositif selon la revendication 13,
**caractérisé en ce que**
les robinets à voies multiples (38 ; 40 ; 42 ; 44 ; 46 ; 48 ; 50 ; 52) peuvent être manoeuvrés par des moteurs d'entraînement contrôlés par la commande (36).

15. Dispositif selon la revendication 14,
**caractérisé en ce que**
la commande (36) est une commande pouvant être programmée électroniquement.
